# EUROPEAN PATENT APPLICATION

(11) **EP 1 384 404 A1**
(43) Date of publication of application: **28.01.2004**
(21) Application number: 02255162.6
(22) Date of filing: 23.07.2002
(51) Int. Cl.: A01N 63/02, A01N 43/16, A01N 43/08, A61K 7/075

(54) **Hair care compositions**

(71) Applicant: THE PROCTER & GAMBLE COMPANY, Cincinnati, Ohio 45202 (US)
(72) Inventor: Konig, Axel, 1780 Wemmel (BE); Ripley, Mark Brian, Middlesex TW2 5AJ (GB); Davison, Gordon Robert, Berks RG42 2QQ (GB)
(74) Representative: Brooks, Maxim Courtney

(57) **Abstract**

A hair-care composition comprising an anti-dandruff effective amount of anti-microbial oligoaminosaccharide comprising at least about 50%, preferably at least about 80% by weight of oligoaminosacharides having from 1 to 50 monomer units. The invention also relates to the use of the anti-microbial oligoaminosaccharide in a hair-care composition for providing anti-dandruff activity.

## Description

### TECHNICAL FIELD

The present invention relates to hair-care compositions, especially to compositions comprising anti-microbial oligoaminosaccharides and uses thereof. The compositions provide superior anti-dandruff benefits.

### BACKGROUND OF THE INVENTION

Anti-dandruff compositions, especially shampoos, are well known in the art and commercially available. Among the preferred type of anti-dandruff agents are particulate, crystalline anti-dandruff materials, such as heavy metal salts of pyridinethione. These salts have been viewed with caution as they can add to the heavy metal load in the environment. Other particulate anti-dandruff agents are sulfur and selenium disulfide. Soluble anti-dandruff agents, such as ketoconazole, are also known in the art.

Efforts have been made to find more environmentally acceptable anti-dandruff agents. WO 97/09961 discloses the use of cationic biopolymers, especially of the chitosan type, as active substances to produce anti-dandruff benefits and to destroy yeast. Traditional chitosan is usually semi-crystalline and only soluble in acidic medium, typically in a pH range from 1 to 5; this limits homogenous formulation and bioavailability. Another drawback of chitosan is that it is difficult to manufacture it with reproducible quality.

The use of cationic polymers in shampoos for conditioning benefits it is also known in the art. JP-A-62-138,418 discloses a shampoo composition comprising anionic surfactant and at least one water-soluble compound derived from chitin.

There is still need for an anti-dandruff composition having superior efficacy as well as a good environmental profile.

### SUMMARY OF THE INVENTION

According to a first aspect of the invention there is provided a hair-care composition that provides superior anti-microbial, especially anti-dandruff efficacy. The composition comprises an anti-dandruff effective amount of an anti-microbial oligoaminosaccharide comprising at least about 50%, preferably at least about 80% by weight of oligoaminosaccharides (sometimes referred herein as oligomers) oligomers components of oligoaminosacharides having from 1 to 50 monomer units. An anti-dandruff effective amount is considered to be more than 0.001% by weight of the composition. In a preferred embodiment the oligoaminosaccharide is present in an amount of from about 0.005% to about 10%, preferably from about 0.01% to about 4% and more preferably from about 0.1% to about 2% by weight of the composition.

In preferred embodiments the hair-care composition additionally comprises from about 0.1% to about 50%, preferably from about 0.5% to about 30%, more preferably from about 1 to about 25% by weight of the composition of a surfactant. The surfactant can be selected from anionic, non-ionic, cationic, amphoteric and zwitterionic surfactants and mixtures thereof.

When the surfactant of the composition is an anionic surfactant the level is preferably from about 5% to about 25%, more preferably from about 10% to about 20% by weight of the composition. Without being bound by the theory, it is believed that optimum anti-microbial performance of the oligoaminosaccharide in a composition comprising anionic surfactant is obtained if the oligoaminosaccharide is solubilised in the composition. A good solubility profile is obtained when the anionic surfactant comprises a mixture of alkyl sulfate and alkyl ether sulfate surfactants. Thus, according to another embodiment of the invention, the hair-care composition of the invention comprises a mixture of an alkyl sulfate and an alkyl ether sulfate surfactant wherein the alkyl sulfate and alkyl ether sulfate surfactants are in a ratio of from about 0.3 to about 0.8. Compositions comprising anionic surfactants can be used, for example, as shampoo compositions. They not only provide excellent anti-dandruff activity but also a very good cleaning profile. In a preferred embodiment the composition of the invention has a pH (as measured at 20°C) of from about 6 to about 7, preferably from about 6.3 to about 6.8, this pH range provides optimum deposition and substantivity of the oligoaminosaccharide and therefore optimum anti-microbial performance.

When the surfactant of the composition is a non-ionic surfactant the level is preferably from about 0.1% to about 5%, more preferably from about 0.1% to about 3% by weight of the composition. Compositions of this kind present optimum anti-dandruff efficacy. Without being bound by theory the anti-dandruff activity is believed to be higher than in other compositions due to the lack of interaction between the oligoaminosaccharide and the non-ionic surfactant.

When the surfactant of the composition is a cationic surfactant the level is preferably from about 0.5% to about 10%, more preferably from about 1 to about 5% by weight of the composition. Compositions of this kind can present not only a good anti-dandruff activity but also a good hair conditioning profile.

In preferred embodiments the anti-microbial oligoaminosaccharide of the composition of the invention comprises at least about 50%, preferably at least about 80% by weight of oligomer components having from 2 to 20 monomer units, preferably from 3 to 12 monomer units.

The term oligoaminosaccharide as used herein includes not only oligoaminosaccharides obtained from natural sources but also synthetically produced oligoaminosaccharides and derivatives thereof of equivalent structure to natural oligoaminosaccharides. The term also covers mixtures of oligomer components as well as the individual oligomers.

It is already known that certain aminosaccharides can provide a degree of anti-dandruff activity. However, the efficacy of aminosaccharides in oligomer form (i.e., less than 50 monomer units), especially chitosan oligomers, has been found to be superior to that of aminosaccharides in other forms, such as for example aminosaccharides in high molecular weight polymer form (i.e., more than 50 monomer units).

Usually oligoaminosaccharides are obtained by various processes involving breaking down the corresponding polymers. The product of these processes is generally a mixture comprising a distribution of oligomers with different number of units and consequently of different molecular weight.

In preferred embodiments the oligoaminosaccharide comprises at least about 40%, preferably at least about 50% by weight of oligoaminosaccharide having from 3 to 10 and preferably from 3 to 5 monomer units. In other preferred embodiments the oligoaminosaccharide comprises at least about 50%, preferably at least about 80% by weight of oligomer components having from 1 to 50, preferably from 2 to 20 and more preferably from 3 to 12 monomer units with less than about 2% by weight of the oligoaminosaccharide having more than 10 monomers. Oligoaminosaccharides having these distributions deliver excellent anti-dandruff benefits. The oligoaminosaccharides used in the compositions of the present invention can also be characterised by having a weight average molecular weight of from about 300 to about 10,000, preferably from about 400 to about 2000.

The oligoaminosaccharides suitable for use herein are preferably soluble at ambient temperature (20°C) in aqueous solutions buffered (using for example acetate or one of the other primary pH standars of DIN 19266) to a pH from about 1 to about 10, preferably from 1 to 12. This solubility range gives the oligoaminosaccharides a great formulation flexibility.

The anti-microbial activity of the oligoaminosaccharide can be evaluated by measuring the Minimum Inhibitory Concentration (MIC) value for a given micro-organism. The anti-dandruff activity can be evaluated, for instance, by measuring the MIC for Malassezia species, preferably *Malassezia furfur.* The oligoaminosaccharide used in the composition of the invention has a MIC for *Malassezia furfur* at 24 hours of less than about 20 ppm, preferably less than about 10 ppm and more preferably less than about 5 ppm. MIC is determined as described hereinbelow. The oligoaminosaccharides not only have a very low MIC values but also have a very fast killing time, preferably the MIC value at 0.5 hours is less than about 50 ppm.

Preferred oligoaminosaccharides for use in the composition of the invention are selected from oligomers of chitosan (including isomeric modified forms), chitosan derivatives and mixtures thereof. Chitosan is a chitin derivative obtained by deacetylation of chitin. Chitin is the main constituent in the shells of crustaceans and is the most abundant naturally occurring biopolymer other than cellulose. Chitosan oligomers can be produced from ordinary commercial chitosan (primary chitosan) by a variety of chemical and physical processes, e.g. acid or base catalysed hydrolysis, enzymatic hydrolysis or by introduction of sonication energy to break the primary chitosan polymer into smaller oligomer fragments. Preferred chitosan oligomers for use herein is COS-Y LDA available from Primex. Chitosan oligomers not only present excellent anti-dandruff activity but also have a safe environmental profile. Low degree of acetylation is preferred for anti-dandruff efficacy. Chitosan oligomers for optimum anti-dandruff activity preferably have a degree of acetylation of less than about 30%, preferably less than about 20%, more preferably less than about 10% and even more preferably less than about 5%. The chitosan oligomers used herein can also provide hair conditioning benefits.

In a preferred embodiment, the composition of the invention comprises the oligoaminosaccharide, especially chitosan oligomers and a suspension of a particulate anti-dandruff agent, especially heavy metal salts of pyridinethione, such as zinc pyridinethione. In another preferred embodiment, the composition of the invention comprises the oligoaminosaccharide, especially chitosan oligomer and a high molecular weight chitosan having at least 50, preferably at least 100 monomer unit on average such as Nanochitosan. The compositions of the invention based on mixtures of anti-dandruff or anti-microbial agents can deliver broad spectrum anti-microbial performance.

In another preferred embodiment the composition of the invention further comprises a hair conditioning agent.

Examples of products comprising the composition of the invention are shampoos, conditioners, hair gels, tonics, mousses, creams, leave-on-products, stylising products and hair sprays.

According to another aspect of the present invention there is provided the use of an anti-microbial oligoaminosaccharide comprising at least about 50%, preferably at least about 80% by weight of oligomer components having from 1 to 50 monomer units in a hair care composition for providing anti-dandruff activity. Optimum anti-dandruff activity has been found when the oligoaminosaccharide comprises at least about 50%, preferably at least about 80% by weight of oligomer component having from 1 to 50, preferably from 2 to 20 and more preferably from 3 to 12 monomer units with less than about 2% by weight of the oligoaminosaccharide having more than 10 monomers. Oligoaminosaccharides having these distributions deliver excellent anti-dandruff benefits. These oligoaminosaccharides are also characterised by having a weight average molecular weight of from about 300 to about 10,000, preferably from about 400 to about 2,000.

In a preferred embodiment the oligoaminosaccharide used for its anti-dandruff activity is soluble in aqueous solutions buffered to a pH of from about 1 to about 10, preferably from 1 to 12 and have a MIC value for *Malassezia furfur* at 24 hours of less than about 20 ppm, preferably less than about 10 ppm and more preferably less than about 5 ppm and preferably a MIC value at 0.5 hours of less than about 50 ppm.

Preferred for use as anti-dandruff agent are oligoaminosaccharides selected from oligomers of chitosan (including isomeric modified forms), chitosan derivatives and mixtures thereof. Preferred for their anti-dandruff activity are chitosan oligomers having a degree of acetylation of less than about 30%, preferably less than about 20%, more preferably less than about 10% and even more preferably less than about 5%.

### DETAILED DESCRIPTION OF THE INVENTION

The present invention envisages hair-care compositions comprising a surfactant and an anti-microbial oligoaminosaccharide. These compositions have superior anti-dandruff properties over other compositions comprising other known anti-dandruff agents. The invention also envisages the use of oligoaminosaccharides for providing anti-dandruff benefits.

### A. Oligoaminosaccharide

Oligoaminosaccharides are those oligomers based on C₄ to C₅ sugars comprising amine groups and derivatives thereof. The oligoaminosaccharides may be composed of one type of sugar or of more than one type. The amines can be primary, secondary, tertiary or quaternary amines depending upon the particular species and the selected pH of the composition. The sugar chain can be straight or branched. Preferably, all of the monomer units may have amine groups attached thereto. Examples of oligoaminosaccharides based on sugars include amino modified celluloses, hydroxyalkylcelluloses, starches, hydroxyalkylstarches, oligomers based on arabinose, xylose, fructose, galactose, manose and mixtures thereof.
The preferred oligoaminosaccharides for use herein are oligomers of 2-amino-2-deoxy-b-D-glucopyranose known as chitosan oligomers.

### MIC test

This test is performed in order to evaluate the anti-microbial activity of the oligoaminosaccharides. A doubling dilution series of the oligoaminosaccharide is prepared and tested against the corresponding micro-organism. MIC is the highest dilution showing no growth. The test is performed in the following manner:
1. Solutions of given concentrations of an oligoaminosaccharide in sterile water buffered to pH 5.5 with lactic acid are prepared.
2. Aliquots (50 µl) of these sterile solutions are dispensed into individual well microtitre plates.
3. A suspension of the corresponding micro-organism (for example *Malassezia furfur)* in peptone is prepared from a 48 hours agar culture.
4. A 15 µl aliquot of the suspension of step 3 is added to each of the well microtitre plates containing the aliquot solutions of step 2 in order to give an initial inoculum of approximately 10⁶ cfu/ml.
5. Steps 1 to 4 are repeated in triplicate for reproducibility purposes.
6. Positive controls containing only lactate acidified water and inoculum (no active) are also prepared.
7. Negative controls containing active solutions only (no inoculum) are prepared to ensure sterility of the solutions.
8. The microtitre plates are incubated at 35°C.
9. Following required incubation time (24 hours) a 0.5 ml aliquot from each dilution is taken and plated onto Dixon Agar.
10. The plates are incubated at 35°C for a minimum of 72 hours and then assessed for growth and the MIC is determined.

### Oligomer distribution and molecular weight

The ratio between oligomers of different molecular weight can be determined by GPC. GPC is performed using Waters chromatograph provided with RI and UV detectors and Ultrahydrogel 125 (300x7.8mm) GPC column calibrated with pululane standards. Elution is performed with ammonium acetate buffering solution containing 0.5M of acetic acid and 0.5M of ammonium acetate pH 4.3 at 20°C with flow rate 0.5 ml/min. Sample concentration is 2mg/ml. Calculations are performed using Waters Millennium software modified for calculation of low resolution signals. Molecular weights of the high Mw admixtures can be estimated according to the formula LgMw = 11.6 - 1.01V(el). Where is V(el) is elution volume.

### B. Surfactant

Surfactants suitable herein include anionic surfactants such as alkyl sulfates, alkyl ether sulfates, alkyl benzene sulfonates, alkyl glyceryl sulfonates, alkyl and alkenyl sulphonates, alkyl ethoxy carboxylates, N-acyl sarcosinates, N-acyl taurates and alkyl succinates and sulfosuccinates, wherein the alkyl, alkenyl or acyl moiety is C₅-C₂₀ , preferably C₁₀-C₁₈ linear or branched; cationic surfactants such as chlorine esters (US-A-4228042, US-A-4239660 and US-A-4260529) and mono C₆-C₁₆ N-alkyl or alkenyl ammonium surfactants wherein the remaining N positions are substituted by methyl, hydroxyethyl or hydroxypropyl groups; low and high cloud point nonionic surfactants and mixtures thereof including nonionic alkoxylated surfactants (especially ethoxylates derived from C₆-C₁₈ primary alcohols), ethoxylated-propoxylated alcohols (e.g., BASF Poly-Tergent® SLF18), epoxy-capped poly(oxyalkylated) alcohols (e.g., BASF Poly-Tergent® SLF18B - see WO-A-94/22800), ether-capped poly(oxyalkylated) alcohol surfactants, and block polyoxyethylene-polyoxypropylene polymeric compounds such as PLURONIC®, REVERSED PLURONIC®, and TETRONIC® by the BASF-Wyandotte Corp., Wyandotte, Michigan; amphoteric surfactants such as the C₁₂-C₂₀ alkyl amine oxides (preferred amine oxides for use herein include C₁₂ lauryldimethyl amine oxide, C₁₄ and C₁₆ hexadecyl dimethyl amine oxide), and alkyl amphocarboxylic surfactants such as Miranol™ C2M; and zwitterionic surfactants such as the betaines and sultaines; and mixtures thereof. Surfactants suitable herein are disclosed, for example, in WO 00/66081.

### C. Conditioning agent

The compositions of the present invention may comprise from about 0.01% to about 10%, by weight of the composition, preferably from about 0.1% to about 8%, more preferably from about 0.1% to about 5%, most preferably from about 0.2% to about 3.5%, of a conditioning agent suitable for application to the hair or skin. It is believed that the conditioning agent provides improved conditioning benefits to the hair, particularly clean hair feel and wet rinse feel.
The conditioning agent may comprise a water insoluble, water dispersible, nonvolatile, liquid that forms emulsified, liquid particles or are solubilized by the surfactant micelles, in the surfactant component. Suitable conditioning agents for use in the present compositions are those conditioning agents characterized generally as silicones (e.g. silicone oils, cationic silicones, silicone gums, high refractive silicones, and silicone resins), organic conditioning oils (e.g. hydrocarbon oils, polyolefins, and fatty esters) or combinations thereof, or those conditioning agents which otherwise form liquid, dispersed, particles in the aqueous surfactant matrix herein. Such conditioning agents should be physically and chemically compatible with the essential components of the composition, and should not otherwise unduly impair product stability, aesthetics or performance. A detailed description of those conditioning agents can be found in Procter and Gamble case 7543.

### D. Other conditioning agents

Also suitable for use in the compositions herein are the conditioning agents described in U.S-A-5,674,478, US-A-5,750,122, US-A-4,529,586, US-A-4,507,280, US-A-4,663,158, US-A-4,197,865, US-A-4,217,914, US-A-4,381,919, and US-A-4,422,853.

Some other preferred silicone conditioning agents for use in the compositions of the present invention include: Abil® S 201 (dimethicone/sodium PG-propyldimethicone thiosulfate copolymer), available from Goldschmidt; DC Q2-8220 (trimethylsilyl amodimethicone) available from Dow Corning; DC 949 (amodimethicone, cetrimonium chloride, and Trideceth-12), available from Dow Corning; DC 749 (cyclomethicone and trimethylsiloxysilicate), available from Dow Corning; DC2502 (cetyl dimethicone), available from Dow Corning; BC97/004 and BC 99/088 (amino functionalized silicone microemulsions), available from Basildon Chemicals; GE SME253 and SM2115-D2_and SM2658 and SF1708 (amino functionalized silicone microemulsions), available from General Electric; siliconized meadowfoam seed oil, available from Croda; and those silicone conditioning agents described by GAF Corp. in US-A-4,834,767 (quaternized amino lactam), by Biosil Technologies in U.S US-A-5,854,319 (reactive silicone emulsions containing amino acids), and by Dow Coming in US-A-4,898,585 (polysiloxanes).

### E. Anti-dandruff particulate

In addition to the anti-microbial oligoaminosaccharide, the compositions of the present invention can comprise one or more anti-dandruff particulates suitable for application to the hair or skin. The anti-dandruff particulate provides the compositions additional anti-microbial activity. Suitable examples of anti-dandruff particulates include: pyridinethione salts, selenium sulfide, particulate sulfur, and mixtures thereof. Preferred are pyridinethione salts. Preferred pyridinethione salts include those formed from metals such as zinc, tin, cadmium, magnesium, aluminum and zirconium, preferably zinc, more preferably the zinc salt of 1-hydroxy-2-pyridinethione (known as "zinc pyridinethione" or "ZPT"), most preferably 1-hydroxy-2-pyridinethione salts in platelet particle form, wherein the particles have an average size of up to about 20µm, preferably up to about 5µm, most preferably up to about 2.5µm. Salts formed from other cations, such as sodium, may also be suitable. Pyridinethione anti-dandruff agents are described, for example, in US-A-2.809.971; US-A-3,236,733; US-A-3,753,196; US-A-3,761,418; US-A-4,345,080; US-A-4,323,683; US-A-4,379,753; and US-A-4,470,982. It is contemplated that when ZPT is used in the compositions herein, the growth or re-growth of hair may be stimulated or regulated, or both, or that hair loss may be reduced or inhibited, or that hair may appear thicker or fuller.

Selenium sulfide is generally regarded as a compound having one mole of selenium and two moles of sulfur, although it may also be a cyclic structure that conforms to the general formula SeₓS_{y}, wherein **x** + **y** = 8. Average particle diameters for the selenium sulfide are typically less than 15µm, as measured by forward laser light scattering device (e.g. Malvern 3600 instrument), preferably less than 10 µm. Selenium sulfide compounds are described, for example, in US-A-2,694,668; US-A-3,152,046; US-A-4,089,945; and US-A-4,885,107.

Such anti-dandruff particulate should be physically and chemically compatible with the essential components of the composition, and should not otherwise unduly impair product stability, aesthetics or performance.

Another anti-dandruff agent, especially suitable in conditioner compositions can be selected from salicylic acid, it salts and mixtures thereof.

### F. Nanochitosan

Other anti-dandruff agents suitable for use in combination with anti-microbial oligoaminosaccharides include Nanochitosan, i.e., chitosan particles with dimensions in the nano range. Suitable chitosan in nanoparticulate form is described in WO 01/32751. Nanochitosan can be made stating from primary chitosan according to the following two-step process.

### a) Dissolution of chitosan in an acidic solution:

1980g of deionised water are weighed into 10 l stainless steel vessel of 22 cm diameter (T). Stirring is started using and overhead stirrer (Heidolph RZR 2041) with 4-pitched-blade impeller of 12 cm diameter (D) (D/T = 0.55). Stirring is carried out at 200 rpm (1.3 m/s tip speed). 20 g of chitosan, sourced from Primex having a molecular weight of approximately 150 kDa and degree of acetylation of 15-20% are added slowly to the vessel, whilst stirring, avoiding contact with the vessel walls or the impeller shaft. Before proceeding to the next stage the mixture is stirred for 5 minutes to allow suspended chitosan to wet. Then, 333 g of an 18% by weight of a lactic acid aqueous solution are added to the vessel to solubilise the chitosan. The resulting mixture is stirred at 200 rpm and maintained for further 10 minutes to allow the chitosan to be fully solubilised in the acidic solution. The resulting solution is filtered through a single 60cm x 60cm layer of mercerised cotton (pre-rinsed in deionised water) fitted in a 6 litres polypropylene Buchner funnel, and the filtrate is collected in a 5 litres clean Buchner flask. This stage removes any insoluble contaminants.

### b)Neutralization of the resulting acidic solution:

The filtered acidic chitosan solution is returned to the 10 1 stainles steel vessel and stirred at 200 rpm. A 1.5% by weight sodium hydroxide aqueous solution is added to the acidic chitosan solution at a rate of 20 ml/min via a peristaltic pump (Watson Marlow 205U) from 2 kg reservoir of 1.5% by weight sodium hydroxide solution. Addition is carried out at 200 rpm until the mixture reaches a pH of 6.85 or 6.90. The vessel content is then mixed at high speed, 600 rpm, for 15 minutes. The stirring speed is reduced to 200 rpm - 250 rpm and 1.5% by weight sodium hydroxide is added at a reduced rate of about 12 ml/min until pH 7.45 - 7.50 reached. The vessel content is mixed at high speed, ∼600rpm (3.8 m/s tip speed) for 15 minutes. Then the stirring speed is reduced to 200 rpm - 250 rpm for 5 minutes to remove air generated in high speed mixing stage. The vessel content is filtered through a single 60 cm x 60 cm layer of mercerised cotton (pre-rinsed in deionised water) fitted in a 6 litres polypropylene Buchner funnel to remove waste liquid. Nanochitosan is insoluble and collects on the cotton layer. Vacuum may be used to speed up removal of waste liquid. Nanochitosan is washed with 3 x 1000 ml aliquots of deionised water. Excess water is removed by vacuum filtration. The washed nanochitosan material is weighed into a tared 1000 ml container. If preservation is required, 1 g of 20% chlorhexidine digluconate is added to container. Deionised water is added to obtain 1000 g total and produce 1 kg of a solution comprising 2% by weight of nano-sized chitosan.

### G. Optional components

Cationic polymers, suspending agents or viscosity modifiers, polyalkylene glycols, hair growth regulating agents, and other optional components are also suitable for the compositions of the invention.

Examples of cationic polymers which may be suitably employed in the compositions herein include copolymers of vinyl monomers, vinyl pyrrolidone copolymers, cationic modified proteins, and certain polymeric quaternary salts.

The anti-dandruff compositions of the present invention may, in some embodiments, comprise from about 0.1% to about 10%, by weight of the composition, preferably from about 0.3% to about 5%, more preferably from about 0.3% to about 2.5%, of a suspending agent suitable for application to the hair or skin. It is believed that the suspending agent suspends water-insoluble, dispersed materials in the compositions. Such suspending agent should be physically and chemically compatible with the essential components of the composition, and should not otherwise unduly impair product stability, aesthetics or performance. Examples of suspending agents which may be suitably employed in especially the shampoo compositions according to the invention include, but are not limited to: acyl derivatives, long chain amine oxides, xanthan gum, carboxyvinyl polymers and mixtures thereof.

Optional viscosity modifiers and thickeners may used, such as: sodium chloride, sodium sulfate, and mixtures thereof.

Examples of other suitable viscosity modifiers for use preferably in conditioners according to the present invention include synthetic hectorites, carboxylic anionic polymers/copolymers and carboxylic anionic cross-linked polymers/ copolymers. Preferred for use herein are carboxylic anionic cross-linked polymers and copolymers. More preferred are carboxylic anionic cross-linked copolymers, such as Carbopol Ultrez 10, available from B.F. Goodrich.

The anti-dandruff compositions of the present invention may, in some embodiments, further comprise from about 0.005% to about 1.5%, by weight of the composition, preferably from about 0.05% to about 1%, more preferably from about 0.1% to about 0.5%, most preferably from about 0.1% to about 0.3%, of selected polyalkylene glycols suitable for application to the hair or skin. The select polyalkylene glycols are believed to provide enhanced lather performance, improved shampoo spreadability, and importantly, increased anti-dandruff particulate efficacy to compositions described herein. Such polyalkylene glycols should be physically and chemically compatible with the essential components described herein, and should not otherwise unduly impair product stability, aesthetics, or performance. Preferred alkylene glycols for use herein comprises polyethylene glycols, polypropylene glycols and mixtures thereof.

The compositions herein may also optionally comprise hair growth regulating agents. Such agents can be chosen from a wide variety of molecules which can function in different ways to enhance the hair growth effects of a compound of the present invention. These optional agents, when present, are typically employed in the compositions herein at a level ranging from about 0.001% to about 15%, preferably from about 0.1% to about 10%, most preferably from about 0.5% to about 5% by weight of the composition.

Other suitable ingredients include: hair conditioning ingredients such as panthenol, panthetine, pantotheine, panthenyl ethyl ether, and combinations thereof; vitamin hair conditioning ingredients such as vitamin E (DL-alpha tocopheryl acetate), vitamin B (such as niacinamide), and mixtures thereof; solvents such as C₁ to C₆ aliphatic alcohol, long chain alcohols and other solvents such as hexylene glycol; hair-hold polymers such as those described in WO-A-94/08557

Other optional components for use in the composition of the invention includes anti-static agents, foam boosters, pH adjusting agents (e.g. sodium citrate, citric acid, succinic acid, phosphoric acid, sodium hydroxide, and sodium carbonate), preservatives (e.g. DMDM hydantoin), anti-microbial agents (e.g. triclosan or triclocarbon), dyes, organic solvents or diluents, pearlescent aids, perfumes, fatty alcohols, proteins, skin active agents, sunscreens, sensates, vitamins, and pediculocides.

Optional anti-static agents such as water-insoluble cationic surfactants may be used, typically in concentrations ranging from about 0.1% to about 5%, by weight of the composition. Such anti-static agents should not unduly interfere with the in-use performance and end-benefits of the shampoo composition; particularly, the anti-static agent should not interfere with the anionic surfactant. A specific non-limiting example of a suitable anti-static agents is tricetyl methyl ammonium chloride.

Optional foam boosters for use in the compositions described herein include fatty ester (e.g. C₈-C₂₂) mono- and di (C₁-C₅, especially C₁-C₃) alkanol amides. Specific non-limiting examples of such foam boosters include coconut monoethanolamide, coconut diethanolamide, and mixtures thereof.

Optional anti-dandruff agents may be used in addition to particulate anti-dandruff actives, typically in concentrations ranging from about 0.1% to about 4%, by weight of the composition, preferably from about 0.2% to about 2%. Such optional anti-dandruff agents include soluble anti-dandruff agents, specific non-limiting examples of which include: piroctone olamine, ketoconazol, and mixtures thereof.

### EXAMPLES

Compositions I to XV exemplify shampoos, compositions XVI to XXI exemplify hair conditioners of the present invention, the compositions present excellent anti-dandruff activity. Levels are given in percentages by weight. As used herein, "minors" refers to those optional components such as preservatives, viscosity modifiers, pH modifiers, fragrances, foam boosters, and the like. As is apparent to the person skilled in the art, the selection of these minors will vary depending on the physical and chemical characteristics of the particular ingredients selected to make the present invention as described herein. The compositions are prepared by conventional methods, the chitosan oligomers are added to the compositions in the form of a 20% by weight active solution, this solution is previously sonicated to achieve total dissolution of the oligomers.

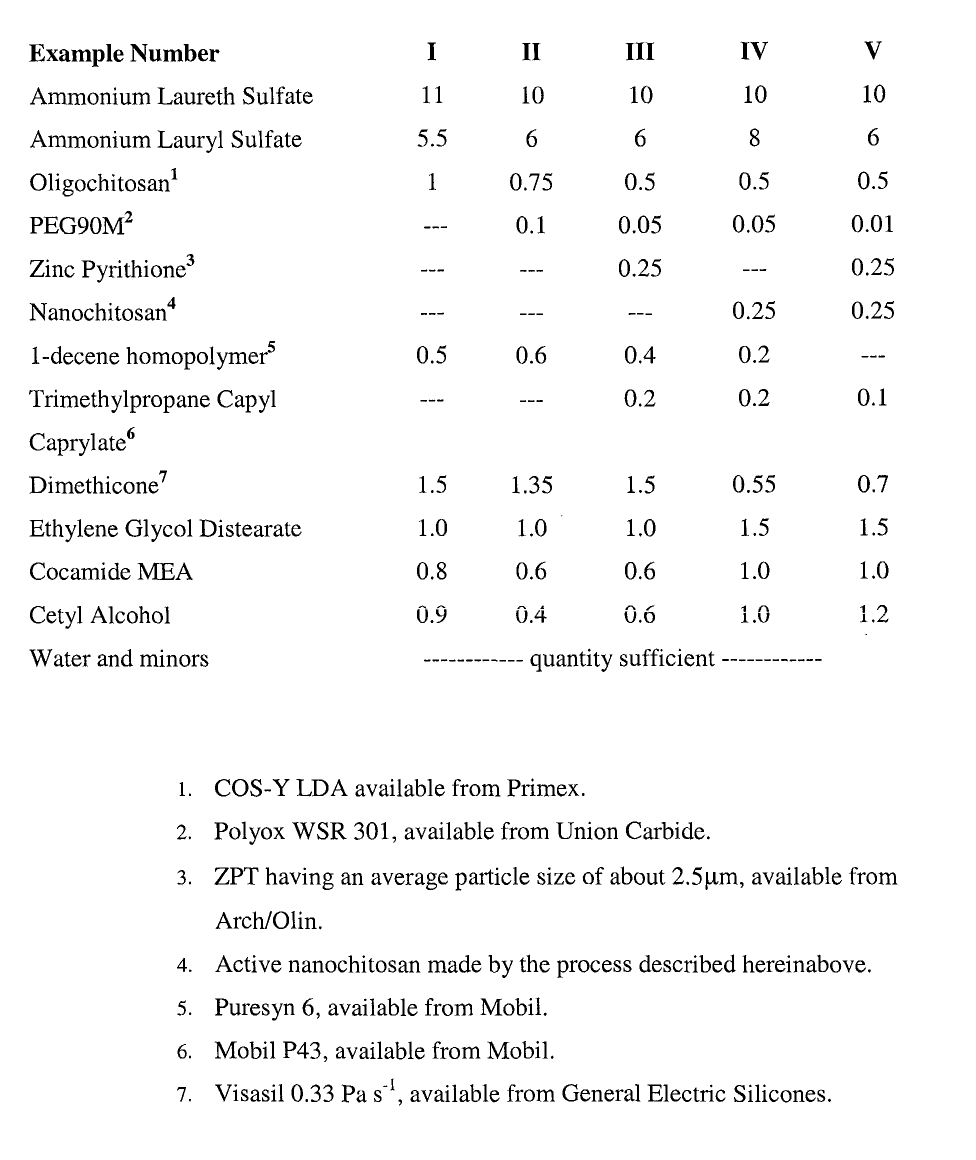

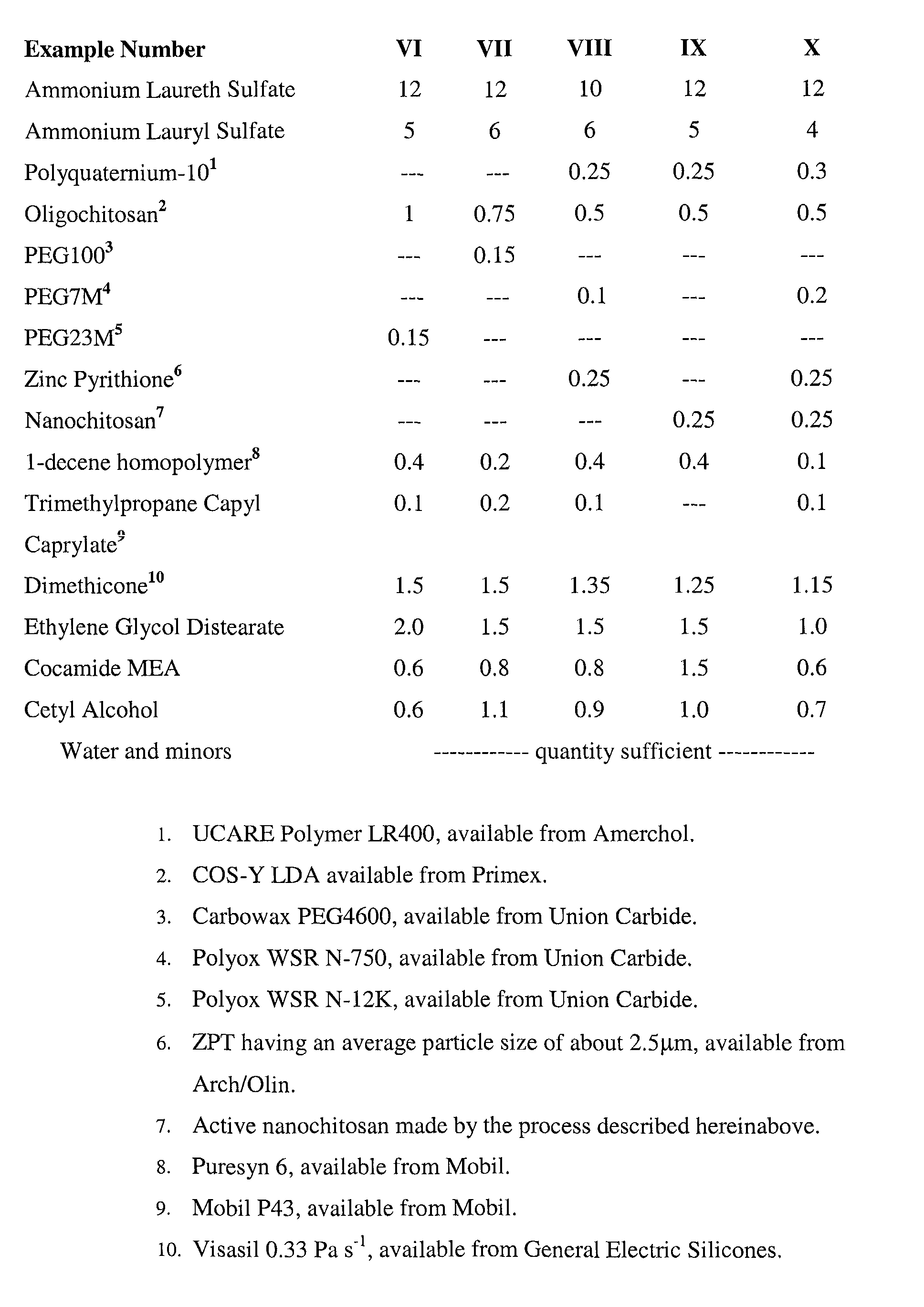

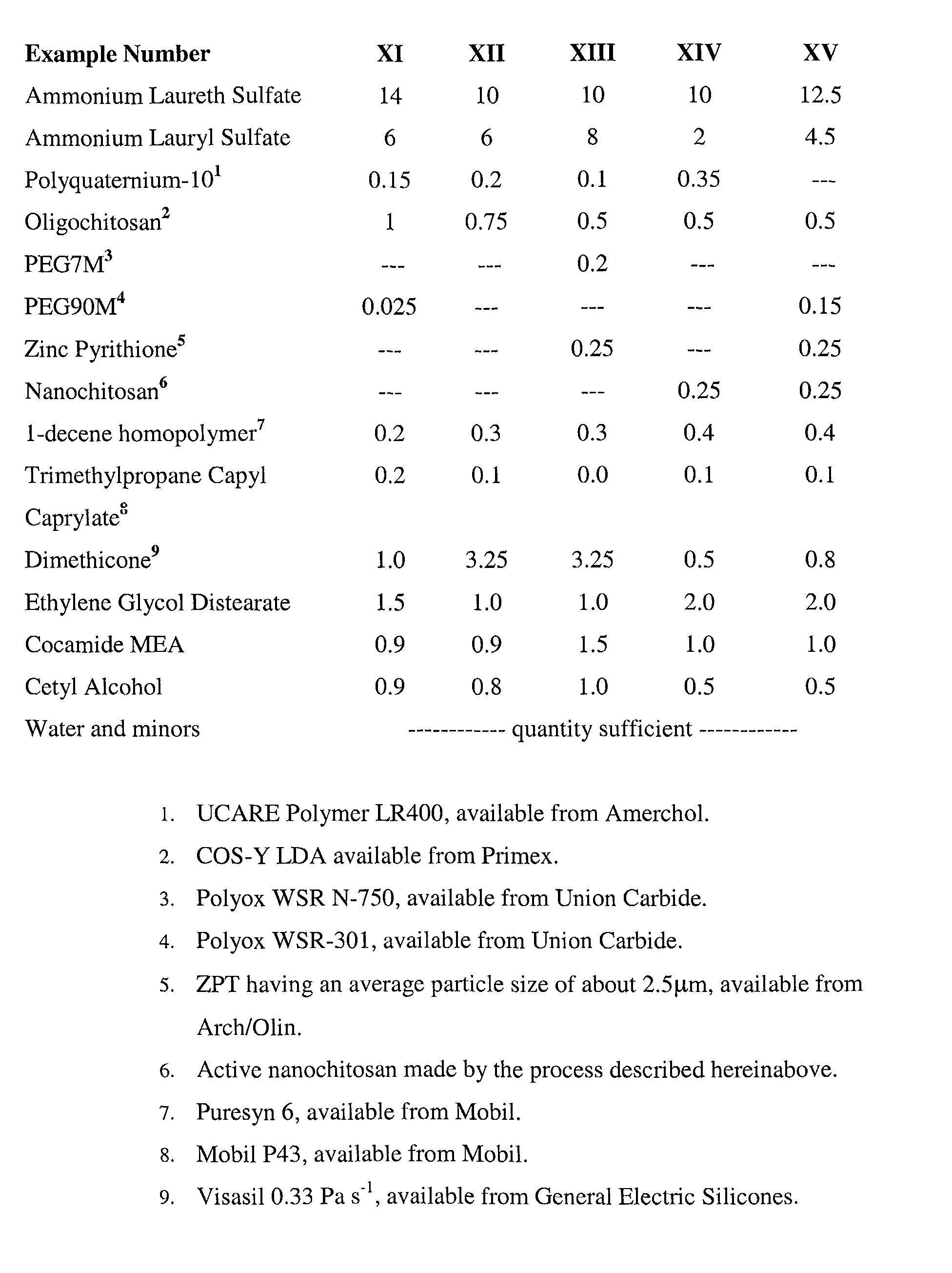

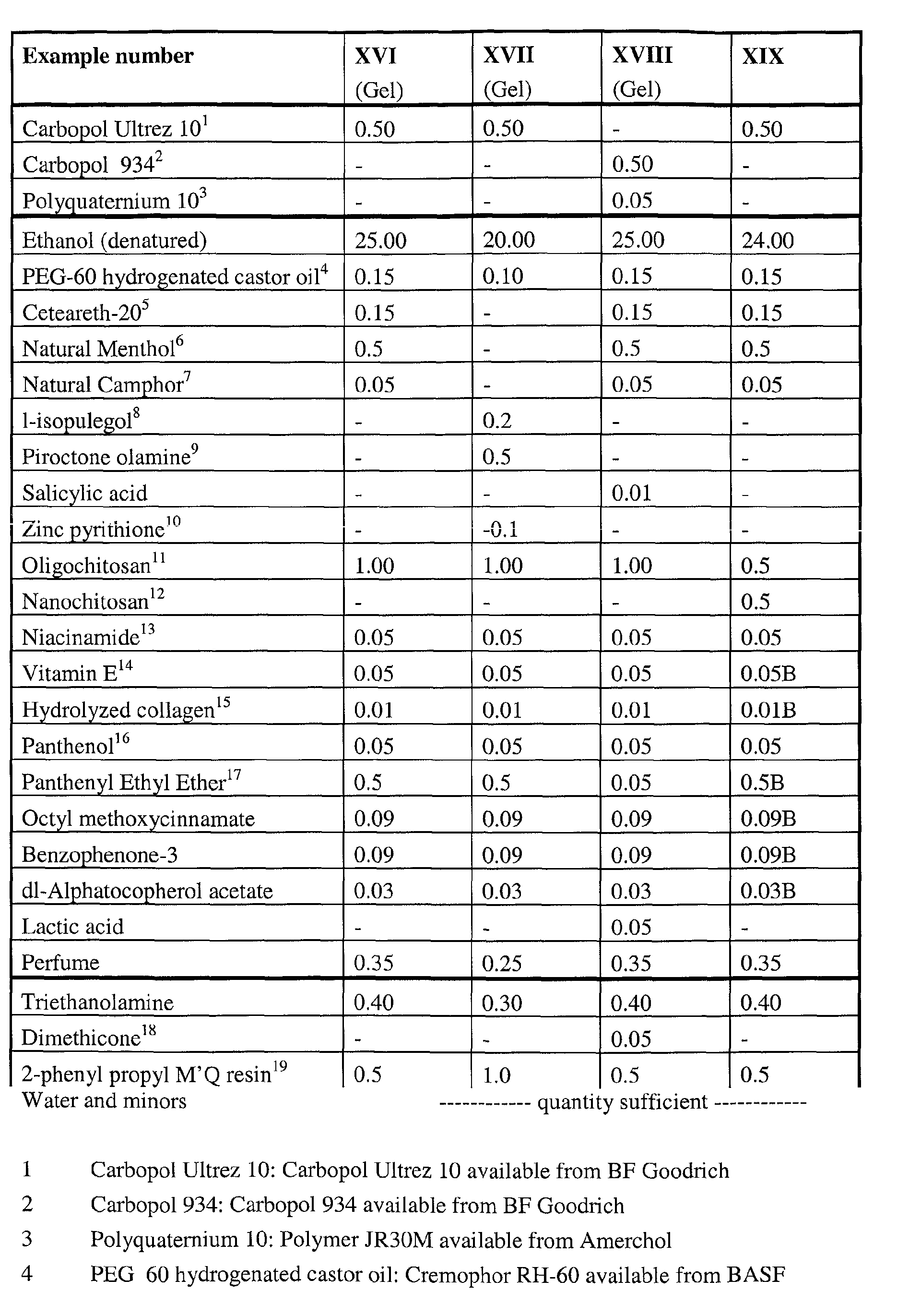

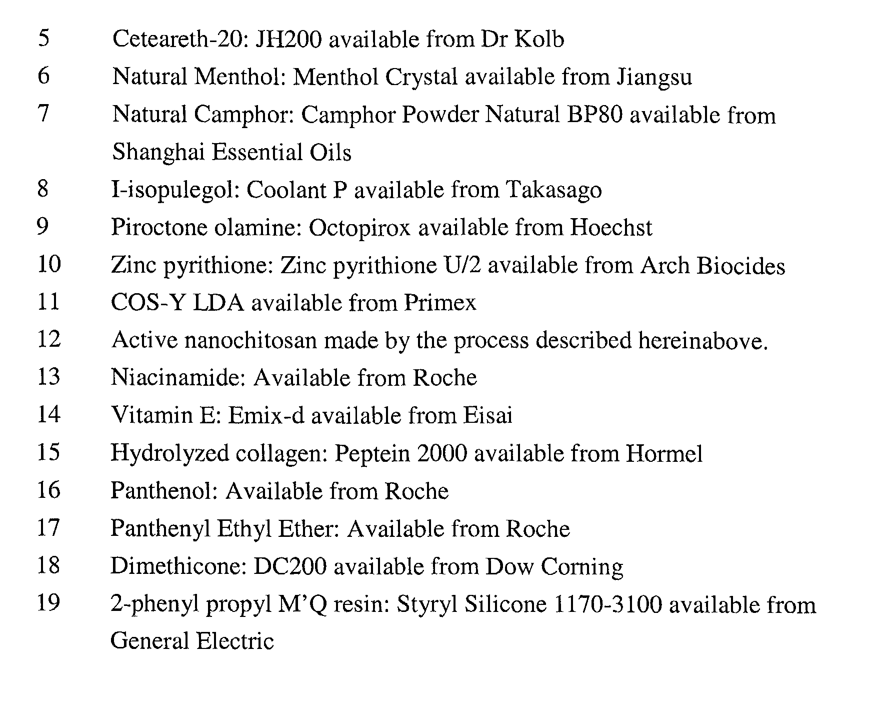

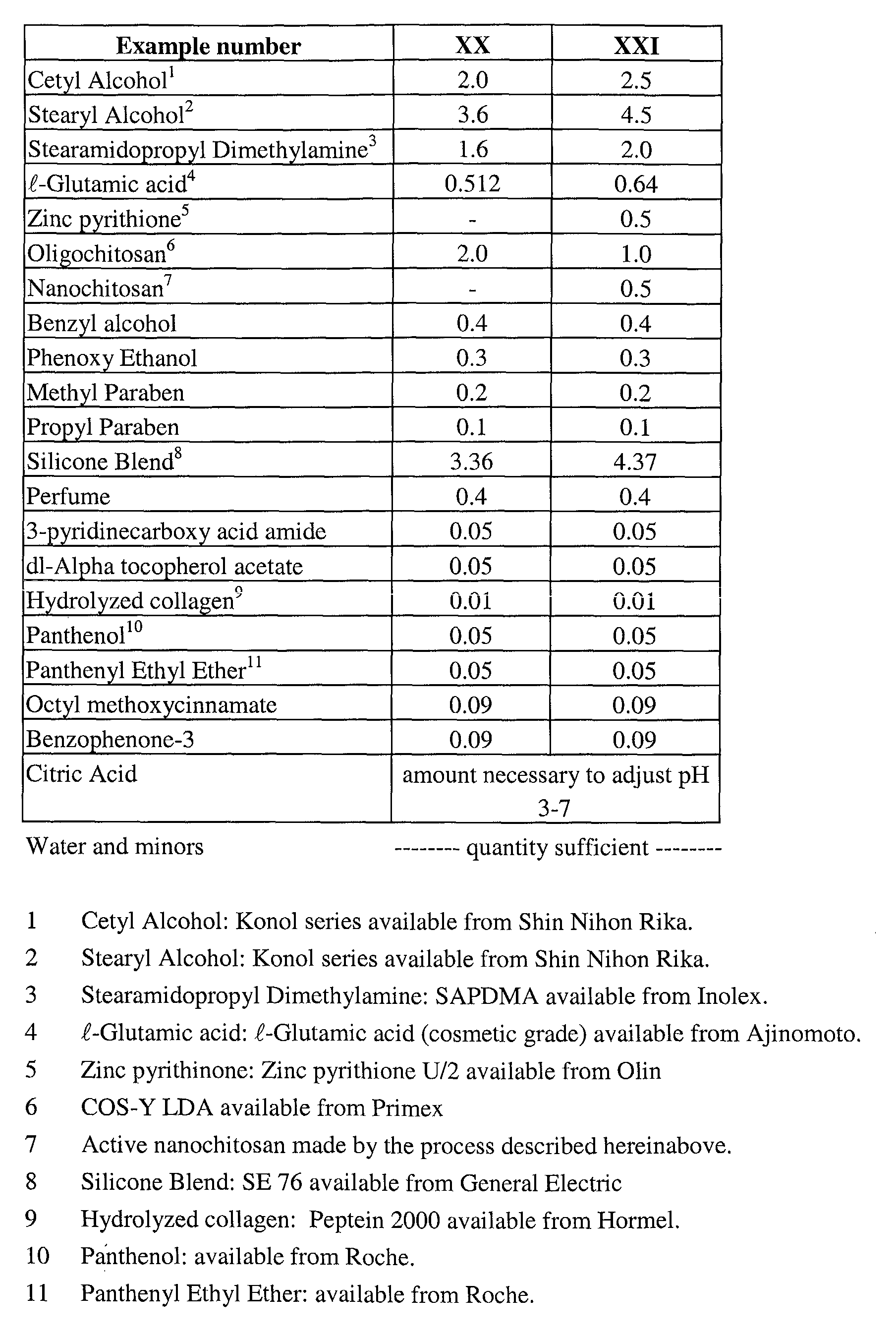

## Claims

1. A hair-care composition comprising an anti-dandruff effective amount of anti-microbial oligoaminosaccharide comprising at least about 50%, preferably at least about 80% by weight of oligoaminosaccharides having from 1 to 50 monomer units.

2. A composition according to claim 1 wherein the oligoaminosaccharide is present in an amount of from about 0.001% to about 10% by weight of the composition.

3. A composition according to claim 1 or 2 additionally comprising from about 0.1% to about 50%, preferably from about 0.5 to about 30% by weight of the composition of a surfactant.

4. A composition according to claim 3 wherein the surfactant is selected from anionic, non-ionic, cationic, amphoteric and zwitterionic surfactants and mixtures thereof.

5. A composition according to claim 4 wherein the surfactant comprises a mixture of alkyl sulfate and alkyl ether sulfate surfactants.

6. A composition according to claim 5 wherein the alkyl sulfate and alkyl ether sulfate surfactants are in a ratio of from about 0.3 to about 0.8.

7. A composition according to any preceding claim in the form of an aqueous solution having a pH of from about 6 to about 7 at 20°C.

8. A composition according to any preceding claim wherein the oligoaminosaccharide comprises at least about 50%, preferably at least about 80% by weight of oligoaminosacharides having from 2 to 20 monomer units.

9. A composition according to any preceding claim wherein the oligoaminosaccharide comprises at least about 50%, preferably at least about 80% by weight of oligoaminosacharides having from 3 to 12 monomer units.

10. A composition according to any preceding claim wherein less than about 2% by weight of the oligomer components has more than 20 monomer units.

11. A composition according to any preceding claim wherein the oligoaminosaccharide has an average molecular weight of from about 300 to about 10,000.

12. A composition according to any preceding claim wherein the Minimum Inhibitory Concentration of the oligoaminosaccharide for *Malassezia furfur* is less than about 20 ppm.

13. A composition according to any preceding claim wherein the oligoaminosaccharide is selected from oligomers of chitosan, chitosan derivatives and mixtures thereof.

14. A composition according to the preceding claim wherein the degree of acetylation of the chitosan or chitosan derivative is less than about 10%, preferably less than about 5%.

15. A composition according to any preceding claim further comprising a suspension of a particulate anti-dandruff agent, preferably a heavy metal salt of pyridinethione.

16. A composition according to any preceding claim further comprising high molecular weight chitosan-derived anti-microbial material, preferably Nanochitosan.

17. A composition according to any preceding claim further comprising a hair-conditioning agent.

18. Use of an anti-microbial oligoaminosaccharide comprising at least about 50%, preferably at least about 80% by weight of oligoaminosacharides having from 1 to 50 monomer units in a hair-care composition for providing anti-dandruff activity.

19. Use of an anti-microbial oligoaminosaccharide according to claim 18 wherein the oligoaminosaccharide is **characterized by** any of the features of claims 8 to 14.
